# EUROPEAN PATENT APPLICATION

(11) **EP 4 215 513 A1**
(43) Date of publication of application: **26.07.2023**
(21) Application number: 21869382.8
(22) Date of filing: 15.09.2021
(51) Int. Cl.: C07C 1/04, C07C 9/00, C01B 3/26, C07B 61/00, C01B 32/40, B01J 23/72, B01J 23/745, B01J 23/80, C25B 1/01

(54) **HYDROCARBON PRODUCTION METHOD**

(30) Priority: 15.09.2020 JP 2020154860
(71) Applicant: ENEOS Corporation, Chiyoda-ku Tokyo 100-8162 (JP)
(72) Inventor: HASHIMOTO Yasushi, Tokyo 100-8162 (JP); IKEDA Masakazu, Tokyo 100-8162 (JP); SUGIURA Yukihiro, Tokyo 100-8162 (JP); KOBAYASHI Atsushi, Tokyo 100-8162 (JP); KAJITA Takuya, Tokyo 100-8162 (JP)
(74) Representative: Algemeen Octrooi- en Merkenbureau B.V.
(86) International application number: PCT/JP2021/033885
(87) International publication number: WO 2022/059696

(57) **Abstract**

This hydrocarbon production method includes a hydrogen extraction step (S10) for extracting hydrogen from an organic hydride via dehydrogenation and a hydrocarbon production step (S12) for producing a hydrocarbon using the extracted hydrogen and carbon monoxide via a reaction carried out according to the Fischer-Tropsch (FT) process. The hydrogen extraction step (S10) utilizes the heat of reaction generated in the hydrocarbon production step (S12).

## Description

### [TECHNICAL FIELD]

The present invention relates to a technique of producing a hydrocarbon.

### [BACKGROUND ART]

As a method of producing a hydrocarbon using hydrogen and carbon monoxide, a Fischer-Tropsch process (hereinafter referred to as "FT process" as appropriate) is known. In addition, as a technique of conveying hydrogen used in the FT process, a technique of conveying hydrogen in the form of an organic hydride such as methylcyclohexane and extracting hydrogen by dehydrogenation is known. This dehydrogenation reaction is an endothermic reaction using a catalyst, and it is necessary to apply heat from the outside in order to maintain the temperature of the catalyst. Therefore, a hydrogen storage/supply system including a heater for heating a catalyst inside a reactor for extracting hydrogen from an organic hydride has been devised (see Patent Literature 1).

### [PRIOR ART DOCUMENT]

### [Patent Literature]

[Patent Literature 1] JP 2003-306301 A

### [SUMMARY OF INVENTION]

### [TECHNICAL PROBLEM]

However, the method of heating the catalyst with the heater described above does not optimize the utilization of heat as a whole system.

The present invention has been made in view of such a situation, and an exemplary object thereof is to provide a new technology for efficiently utilizing heat in the production of hydrocarbons.

### [SOLUTION TO PROBLEM]

In order to solve the above problems, a hydrocarbon production method according to an aspect of the present invention includes: a hydrogen extraction step of extracting hydrogen from an organic hydride by a dehydrogenation reaction; and a hydrocarbon production step of producing a hydrocarbon by a reaction by an FT process using the extracted hydrogen and carbon monoxide. In the hydrogen extraction step, reaction heat generated in the hydrocarbon production step is used.

### [ADVANTAGEOUS EFFECTS OF INVENTION]

According to one aspect of the present invention, heat can be efficiently used in production of a hydrocarbon.

### [BRIEF DESCRIPTION OF DRAWINGS]

Fig. 1 is a block diagram of a hydrocarbon production system according to a first embodiment.
Fig. 2 is a schematic view showing a schematic configuration of the reactor according to the first embodiment.
Fig. 3 is a block diagram of a hydrocarbon production system according to a second embodiment.
Fig. 4 is a block diagram of a hydrocarbon production system according to a third embodiment.
Fig. 5 is a flowchart of the hydrocarbon production method according to each embodiment.

### [DESCRIPTION OF EMBODIMENTS]

First, aspects of the present invention will be listed. A hydrocarbon production method according to an aspect of the present invention includes: a hydrogen extraction step of extracting hydrogen from an organic hydride by a dehydrogenation reaction; and a hydrocarbon production step of producing a hydrocarbon by a reaction by an FT process using the extracted hydrogen and carbon monoxide. In the hydrogen extraction step, reaction heat generated in the hydrocarbon production step is used.

According to this aspect, the reaction heat generated in the hydrocarbon production step can be used as heat required in the dehydrogenation reaction which is an endothermic reaction.

The dehydrogenation reaction vessel in which the dehydrogenation reaction occurs may be provided in a region where the reaction heat generated in the hydrocarbon production step is transferred without passing through the heat medium. As a result, the heat medium is unnecessary, and the configuration of the manufacturing apparatus can be simplified.

As a heat medium for utilizing the reaction heat generated in the hydrocarbon production step in the hydrogen extraction step, a heat medium oil or water vapor may be used. This increases the degree of freedom in layout between the apparatus in which the hydrogen extraction step is performed and the apparatus in which the hydrocarbon production step is performed. By selecting an appropriate heat medium oil, heat can be transferred at a higher temperature. Since water vapor is stable as a heat medium and is also a non-combustible substance, the degree of freedom of the device configuration increases from the viewpoint of safety.

Carbon monoxide may be produced from carbon dioxide recovered from the atmosphere or fuel exhaust gases. This contributes to realization of carbon neutral while producing a hydrocarbon which is a raw material of a synthetic fuel.

The step of producing carbon monoxide may be a reverse shift reaction step of producing carbon monoxide from carbon dioxide by a reverse shift reaction.

The reverse shift reaction step may be performed at 290 to 340°C. As a result, the carbon monoxide produced by the reverse shift reaction can be subjected to the reaction by the FT process without lowering the temperature so much, and the reactor and the process can be simplified.

In the reverse shift reaction step, a copper-based catalyst body containing a copper component composed of at least one of copper or copper oxide may be used, and in the reaction by the FT process, an iron-based catalyst body containing an iron component composed of at least one of iron or iron oxide and an added metal composed of at least one of alkali metal or alkaline earth metal may be used. As a result, it is possible to react the reverse shift reaction and the reaction by the FT process at the same place.

The step of producing carbon monoxide may be an electrolytic reduction step of producing carbon monoxide from carbon dioxide by electrolytic reduction.

In the hydrocarbon production step, a reaction by the FT process may occur in an FT reaction vessel having a fixed bed or a slurry bed.

In the hydrogen extraction step, latent heat of water produced in the hydrocarbon production step may be used. In the hydrogen extraction step, heat obtained by burning the hydrocarbon produced in the hydrocarbon production step may be used. In the hydrogen extraction step, heat of an electric heater driven by electricity generated using the hydrocarbon produced in the hydrocarbon production step may be used. As a result, heat can be efficiently used in the production of hydrocarbon.

Note that optional any combination of the aforementioned constituent elements, or any conversion of the expressions of the present invention among methods, apparatuses, systems, and the like may also effective as an aspect of the present invention. In addition, an appropriate combination of the above-described elements can also be included in the scope of the invention for which patent protection is sought by the present patent application.

Hereinafter, the present invention will be described on the basis of preferred embodiments with reference to the drawings. The embodiments are not intended to limit the invention but are merely examples, and all features described in the embodiments and combinations thereof are not necessarily essential to the invention. The same or equivalent constituent elements, members, and processes illustrated in the drawings are denoted by the same reference numerals, and redundant description will be omitted as appropriate. In addition, the scale and shape of each part illustrated in each drawing are set for convenience in order to facilitate the description, and are not limitedly interpreted unless otherwise specified. In addition, even in the case of the same member, scales and the like may be slightly different between the drawings. In addition, the terms "first", "second", and the like used in the present specification or claims do not represent any sequential order or priority unless otherwise specified, and are intended to distinguish one configuration from another configuration.

### First Embodiment

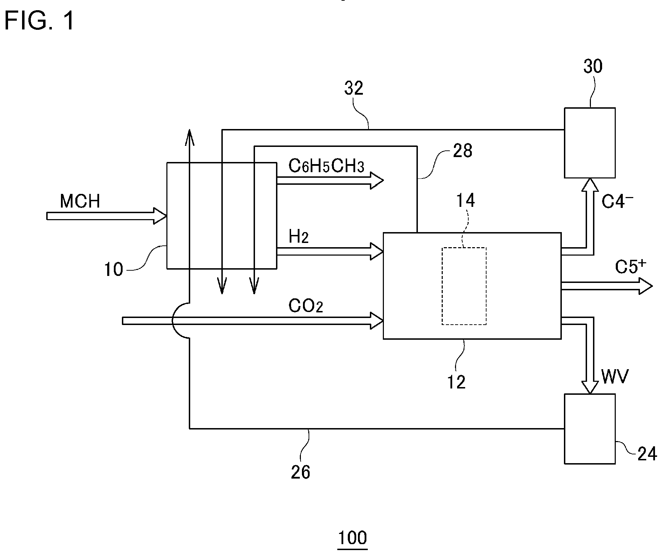

Fig. 1 is a block diagram of a hydrocarbon production system according to a first embodiment. A hydrocarbon production system 100 shown in Fig. 1 includes a dehydrogenation apparatus 10 for extracting hydrogen from an organic hydride by a dehydrogenation reaction, and a hydrocarbon production apparatus 12 for producing a hydrocarbon using hydrogen and carbon monoxide.

The dehydrogenation apparatus 10 generates hydrogen and an aromatic compound from an organic hydride by a dehydrogenation reaction in the presence of a dehydrogenation catalyst. Examples of the organic hydride include decalin, dimethyldecalin, methylcyclohexane, and ethylcyclohexane. Examples of the aromatic compound include naphthalene, dimethylnaphthalene, toluene, and ethylbenzene.

Examples of the dehydrogenation catalyst include a catalyst carrier on which at least one noble metal selected from the group consisting of platinum (Pt), palladium (Pd), and rhodium (Rh) is supported. Preferably, nickel (Ni) may be supported on the catalyst carrier. The reaction temperature of the dehydrogenation catalyst is selected, for example, in the range of 280 to 340°C.

In dehydrogenation apparatus 10 according to the present embodiment, for example, hydrogen and toluene (C₆H₅CH₃) are produced from methylcyclohexane (MCH: C₆H₁₁CH₃) by a dehydrogenation reaction shown in the following formula (1) in a state of being heated to about 300°C.

C₆H₁₁CH₃ → C₆H₅CH₃ + 3H₂ ΔH_{298K} = + 205 kJ/mol... Formula (1)

The dehydrogenation reaction of Formula (1) is an endothermic reaction (ΔH_{298K} is a positive value). The reaction from MCH to toluene and hydrogen is preferably performed under conditions of high temperature and low pressure in terms of chemical equilibrium. Therefore, in order to continue a stable dehydrogenation reaction, it is necessary to supply heat from the outside.

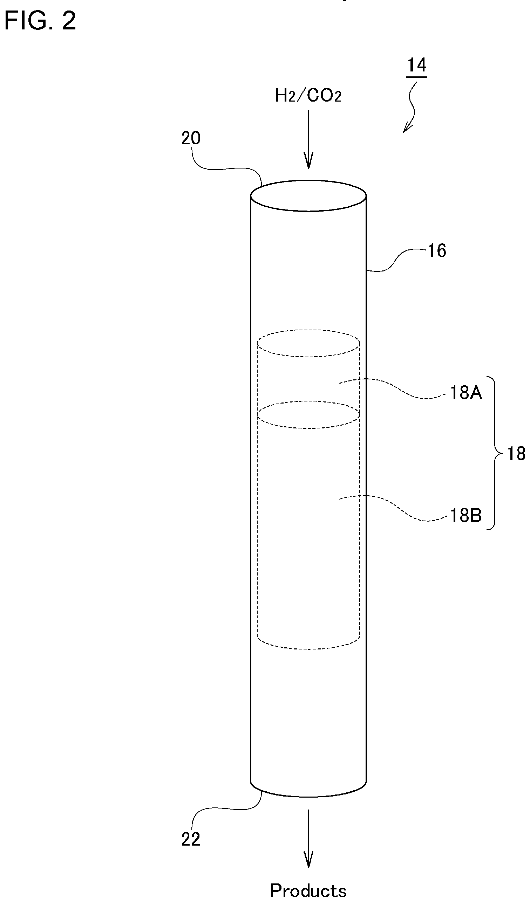

Hydrogen taken out by the dehydrogenation apparatus 10 is supplied to the hydrocarbon production apparatus 12. Carbon dioxide is supplied to the hydrocarbon production apparatus 12 in addition to hydrogen. The hydrocarbon production apparatus 12 is provided with a reactor 14 having a plurality of kinds of catalyst layers. Fig. 2 is a schematic view showing a schematic configuration of the reactor 14 according to the first embodiment. The reactor 14 shown in Fig. 2 is a fixed bed reactor including a reaction vessel 16 which is a cylindrical reaction tube, and a catalyst layer 18 fixed in the reaction vessel 16.

The reaction vessel 16 has a source gas inlet 20 provided at one end and a hydrocarbon outlet 22 provided at the other end. A source gas containing hydrogen and at least one of carbon dioxide or carbon monoxide is introduced from the source gas inlet 20 side. While the source gas flows in the reaction vessel 16 from the source gas inlet 20 toward the hydrocarbon outlet 22, a product containing liquid hydrocarbon is produced in the presence of the catalyst layer 18. The product is discharged from the hydrocarbon outlet 22.

The catalyst layer 18 includes a first catalyst layer 18A containing a copper-based catalyst body and a second catalyst layer 18B containing an iron-based catalyst body. The copper-based catalyst body contains a copper component composed of at least one of copper or copper oxide. The iron-based catalyst body contains an iron component composed of at least one of iron or iron oxide, and an added metal composed of at least one of alkali metal or alkaline earth metal. The first catalyst layer 18A and the second catalyst layer 18B are laminated in this order from the source gas inlet 20 side. The first catalyst layer 18A is formed, for example, by filling a plurality of granular copper-based catalyst bodies in the reaction vessel 16. The second catalyst layer 18B is formed, for example, by filling a plurality of granular iron-based catalyst bodies in the reaction vessel 16.

The form of each catalyst body is not particularly limited, and may be, for example, a powder, and is preferably a granular molded body formed of an aggregate of powder. The shape of the catalyst body which is a granular molded body is not particularly limited, and may be, for example, a cylindrical shape, a prismatic shape, a spherical shape, or an amorphous shape. The particle size (maximum width) of the granular molded body may be 1 mm or more and 50 mm or less. The particle size (maximum width) of the powder of the catalyst body may be 1 um or more and less than 1000 µm.

In order for the copper-based catalyst body to function as a catalyst, at least metallic copper needs to be present in the copper-based catalyst body during the reaction. Therefore, the catalyst is usually subjected to reduction treatment before being used in the reaction. The copper-based catalyst body before the reduction treatment usually contains copper oxide (for example, CuO).

The content of the copper component in the copper-based catalyst body is preferably 20 to 100 mass% based on the mass of the entire copper-based catalyst body when the entire amount of the copper component contained in the copper-based catalyst body is converted into the amount of metal copper.

The copper-based catalyst body may further contain zinc oxide (ZnO). When the copper-based catalyst body contains zinc oxide, liquid hydrocarbon can be more efficiently produced. When the copper-based catalyst body contains zinc oxide, the proportion of the amount of zinc oxide is preferably 10 to 70 mass%, and more preferably 20 to 50 mass%, based on the total amount of copper oxide and zinc oxide, when the entire amount of the copper element contained in the copper-based catalyst body is converted into the amount of copper oxide (CuO).

The copper-based catalyst body may further contain a carrier that supports a copper component. When the copper-based catalyst body contains zinc oxide, zinc oxide is also usually supported on the carrier. The carrier is preferably alumina such as γ-alumina. When the copper-based catalyst body contains a carrier, the content of the carrier in the copper-based catalyst body is, for example, 5 to 60 mass%, preferably 10 to 50 mass%, and more preferably 15 to 40 mass%, based on the total of the content of copper, the content of zinc oxide, and the content of the carrier. Here, the content of copper means an amount obtained by converting all the amounts of copper components contained in the copper-based catalyst into the amount of metallic copper.

The copper-based catalyst body containing a copper component and zinc oxide can be obtained, for example, by a method including a step of generating a precipitate containing copper and zinc by a coprecipitation method and a step of firing the produced precipitate. The precipitate includes, for example, a hydroxide of copper and zinc, a carbonate, or a composite salt thereof. A copper-based catalyst body containing a copper component, zinc oxide, and a carrier is obtained by producing a precipitate containing copper and zinc from a solution containing a carrier (for example, alumina) by a coprecipitation method.

A fired body formed by firing and containing a copper component and zinc oxide may be pulverized, or a granular molded body may be formed by further molding the powder. Examples of the method for molding the powder include extrusion molding and tablet molding. It is also possible to obtain a molded body by molding a mixture containing the powder of the fired body and carbon black.

In order for the iron-based catalyst to function as a catalyst, at least metallic iron needs to be present in the iron-based catalyst body during the reaction. Therefore, the catalyst is usually subjected to reduction treatment before being used in the reaction. The iron-based catalyst body before the reduction treatment usually contains iron oxide (for example, Fe₃O₄).

The content of the iron component in the iron-based catalyst body is preferably 20 to 100 mass% based on the mass of the entire iron-based catalyst body when the entire amount of the iron component contained in the iron-based catalyst body is converted into the amount of metal iron.

The added metal contains one or more kinds optionally selected from an alkali metal and an alkaline earth metal. For example, the added metal preferably contains at least one selected from the group consisting of sodium, potassium, and cesium. When the added metal contains sodium, potassium, or cesium, a liquid hydrocarbon can be more efficiently produced.

The content of the added metal in the iron-based catalyst body is preferably 0.2 to 40 mass%, and more preferably 0.5 to 20 mass%, based on the amount of a portion other than the added metal in the iron-based catalyst boy. When the added metal contains sodium, the content of sodium in the iron-based catalyst body is preferably 0.2 to 20 mass%, and more preferably 0.5 to 10 mass%. When the added metal contains potassium, cesium, or a combination thereof, the total content of potassium and cesium in the iron-based catalyst body is preferably 0.2 to 40 mass%, and more preferably 0.5 to 20 mass%. When the content of the added metal is within the above range, the conversion of carbon dioxide or carbon monoxide tends to be further improved.

The iron-based catalyst body can be obtained, for example, by a method including a step of producing a precipitate of an iron compound containing trivalent iron and divalent iron from an aqueous solution containing Fe³⁺ and Fe²⁺, a step of heating the precipitate to form a heating body containing iron oxide, and a step of attaching an aqueous solution containing an added metal to the heating body and then drying the aqueous solution containing the added metal.

The heating body containing iron oxide may be pulverized, or a granular molded body may be formed by further molding the powder. Examples of the method for molding the powder include extrusion molding and tablet molding. It is also possible to obtain a molded body by molding a mixture containing the powder of the fired body and carbon black.

In the first catalyst layer 18A according to the present embodiment, carbon monoxide is produced from carbon dioxide by a reverse shift reaction represented by the following Formula (2) as a step of generating carbon monoxide.

H₂ + CO₂ → CO + H₂O... Formula (2)

In addition, in the second catalyst layer 18B, as a step of producing a hydrocarbon, a hydrocarbon is produced from carbon monoxide by a reaction by an FT process shown in the following Formula (3).

2nH₂ + nCO → (-CH₂-)ₙ + nH₂O... Formula (3)

Therefore, when n = 1, an exothermic reaction represented by the following Formula (4) occurs as a whole in the reaction vessel 16.

3H₂ + CO₂ → CH₂ + 2H₂O ΔH_{298K} = -111kJ/mol... Formula (4)

In the present embodiment, during the progress of the reaction for producing a hydrocarbon from the source gas, the source gas is pressurized to 2MPa, and catalyst layer 18 is heated to 320°C. The heating temperature for the reaction is, for example, 200 to 400°C. The reverse shift reaction step according to the present embodiment is performed in the range of 290 to 340°C. As a result, the carbon monoxide produced by the reverse shift reaction can be subjected to the reaction by the FT process without lowering the temperature so much, and the reactor and the process can be simplified. In addition, the reverse shift reaction and the reaction by the FT process can be reacted in the same reaction vessel.

Then, the hydrocarbon production apparatus 12 can produce a hydrocarbon C4- having four or less carbon atoms and a liquid hydrocarbon C5+ having five or more carbon atoms in a high yield by the reaction in the included reactor 14. In addition, water vapor WV of 200°C or higher is produced together with the generation of hydrocarbon. Therefore, as shown in Fig. 1, the water vapor WV produced by the hydrocarbon production apparatus 12 may be heated to a reaction temperature of the dehydrogenation catalyst or higher by a heater 24 such as a boiler, and may be used as a heating source during the reaction of the dehydrogenation apparatus 10 via the heat medium line 26. When the reaction temperature of the dehydrogenation catalyst is lower than the water vapor WV, the water vapor WV may be directly used for heating the dehydrogenation apparatus 10 without using the heater 24.

As described above, in the hydrogen extraction step in the dehydrogenation apparatus 10, latent heat of water produced in the hydrocarbon production step may be used.

The reaction heat in the hydrocarbon production apparatus 12 is used as a heating source during the reaction of the dehydrogenation apparatus 10 via the heat medium line 28. At this time, water vapor or heat medium oil may be used as the heat medium.

As described above, the hydrocarbon production system 100 can use the reaction heat generated in the hydrocarbon production step in the hydrocarbon production apparatus 12 as heat necessary for the dehydrogenation reaction in the hydrogen extraction step in the dehydrogenation apparatus 10.

Furthermore, the reactor 14 used in the hydrocarbon production step has the reaction vessel 16 having a fixed bed in which a reaction by the FT process occurs, but may be a reactor vessel having a slurry bed instead of the fixed bed. Carbon dioxide to be supplied to the hydrocarbon production apparatus 12 may be recovered from the atmosphere or combustion exhaust gas.

In addition, the hydrocarbon C4- having 4 or less carbon atoms produced in the hydrocarbon production apparatus 12 may be burned in a combustion device 30, and the obtained heat may be used as a heating source during the reaction of the dehydrogenation apparatus 10 via the heat medium line 32.

Hydrocarbon (for example, methane) produced by hydrocarbon production apparatus 12 may be used to generate power by a fuel cell, and the heat of the electric heater driven by the electricity may be used as a heating source during the reaction of dehydrogenation apparatus 10.

### Second Embodiment

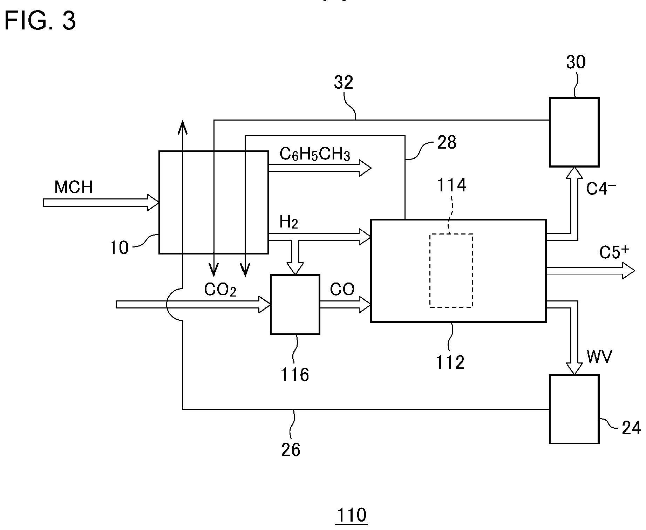

Fig. 3 is a block diagram of a hydrocarbon production system according to a second embodiment. The hydrocarbon production system 110 shown in Fig. 3 is mainly different from the hydrocarbon production system 100 according to the first embodiment in that an apparatus for performing a step of producing carbon monoxide from carbon dioxide in a step prior to the hydrocarbon production apparatus is separately provided. Therefore, in the description of the hydrocarbon production system 110 according to the second embodiment, the same components as those of the hydrocarbon production system 100 according to the first embodiment are denoted by the same reference numerals, and the description thereof will be appropriately omitted.

The hydrocarbon production system 110 includes a carbon monoxide producing apparatus 116 that produces carbon monoxide from carbon dioxide, and a hydrocarbon production apparatus 112 that is supplied with the produced carbon monoxide and produces a hydrocarbon by a reaction by an FT process. The hydrocarbon production apparatus 112 is provided with a reactor 114 having a catalyst layer necessary for the reaction by the FT process.

The carbon monoxide producing apparatus 116 is provided with a reaction vessel including a catalyst layer used for the above-described reverse shift reaction. Then, carbon dioxide supplied from the outside is converted into carbon monoxide based on the reaction of Formula (2) described above. Hydrogen used in the reaction of Formula (2) may be supplied from the dehydrogenation apparatus 10. Note that the carbon monoxide producing apparatus 116 may be an electrolytic reduction apparatus that generates carbon monoxide from carbon dioxide by electrolytic reduction.

The reactor 114 has the second catalyst layer 18B in the first embodiment. In the second catalyst layer 18B, as a step of producing a hydrocarbon, a hydrocarbon is produced from carbon monoxide by a reaction by an FT process shown in the following Formula (5).

2H₂ + CO → (-CH₂-) + H₂O ΔH_{298K} = -152 kJ/mol... Formula (5)

In the present embodiment, during the progress of the reaction for producing a hydrocarbon from the carbon monoxide, the carbon monoxide is pressurized to 2MPa, and the second catalyst layer 18B is heated to 200°C.

As described above, the hydrocarbon production system 110 according to the second embodiment can use the reaction heat generated in the hydrocarbon production step in the hydrocarbon production apparatus 112 as heat necessary for the dehydrogenation reaction in the hydrogen extraction step in the dehydrogenation apparatus 10.

### Third Embodiment

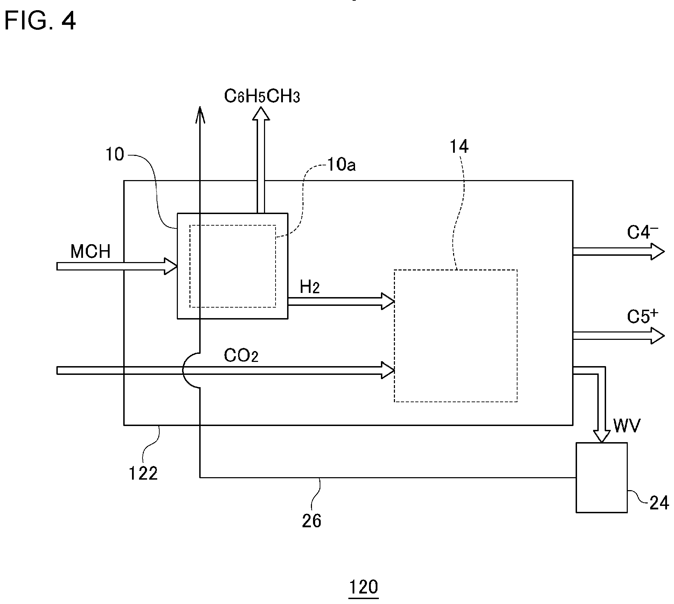

Fig. 4 is a block diagram of a hydrocarbon production system according to a third embodiment. The hydrocarbon production system 120 shown in Fig. 4 is mainly different from the hydrocarbon production system 100 according to the first embodiment in that the reaction vessel 10a in the dehydrogenation apparatus 10 is provided inside the hydrocarbon production apparatus 122. Therefore, in the description of the hydrocarbon production system 120 according to the third embodiment, the same components as those of the hydrocarbon production system 100 according to the first embodiment are denoted by the same reference numerals, and the description thereof will be appropriately omitted.

In the hydrocarbon production system 120 according to the present embodiment, the reaction vessel 10a in which the dehydrogenation reaction occurs is provided inside the hydrocarbon production apparatus 122. That is, the reaction vessel 10a is provided in a region where the reaction heat generated in the reactor 14 provided in the hydrocarbon production apparatus 122 is transferred without passing through the heat medium. As a result, the heat medium is unnecessary, and the configuration of the hydrocarbon production system 120 can be simplified. The region where the heat is conducted without passing through the heat medium is, for example, a region that is in contact with the reactor 14 or a region that has the same atmosphere as that of the reactor 14.

### Hydrocarbon Production Method

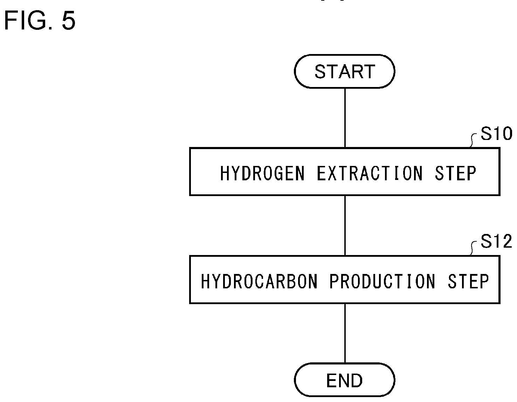

Fig. 5 is a flowchart of the hydrocarbon production method according to each embodiment. As shown in Fig. 5, the hydrocarbon production method according to each embodiment includes: a hydrogen extraction step (S10) of extracting hydrogen from an organic hydride by a dehydrogenation reaction; and a hydrocarbon production step (S12) of producing a hydrocarbon by a reaction by an FT process using the extracted hydrogen and carbon monoxide. In addition, in the hydrogen extraction step (S10), reaction heat generated in the hydrocarbon production step (S12) is used.

Although the present invention has been described with reference to each of the above-described embodiments, the present invention is not limited to each of the above-described embodiments, and configurations obtained by appropriately combining or replacing the configurations of each of the embodiments are also included in the present invention. In addition, it is also possible to recombine the combination and the sequential order of processing in each of the embodiments as appropriate on the basis of the knowledge of those skilled in the art, and to add modifications such as various design changes to the embodiments, and the embodiments to which such modifications are added can also be included in the scope of the present invention.

### [INDUSTRIAL APPLICABILITY]

The present invention can be used in a hydrocarbon production method.

### [REFERENCE SIGNS LIST]

10 dehydrogenation apparatus, 10a reaction vessel, 12 hydrocarbon production apparatus, 14 reactor, 16 reaction vessel, 18 catalyst layer, 18A first catalyst layer, 18B second catalyst layer, 20 source gas inlet, 22 hydrocarbon outlet, 24 heater, 26, 28 heat medium line, 30 combustion device, 32 heat medium line, 100 hydrocarbon production system, 110 hydrocarbon production system, 112 hydrocarbon production apparatus, 114 reactor, 116 carbon monoxide producing apparatus, 120 hydrocarbon production system, 122 hydrocarbon production apparatus

## Claims

1. A hydrocarbon production method including:
a hydrogen extraction step of extracting hydrogen from an organic hydride by a dehydrogenation reaction; and
a hydrocarbon production step of producing a hydrocarbon by a reaction by a Fischer-Tropsch (FT) process using the extracted hydrogen and carbon monoxide, wherein the hydrogen extraction step utilizes reaction heat generated in the hydrocarbon production step.

2. The hydrocarbon production method according to claim 1, wherein a dehydrogenation reaction vessel in which the dehydrogenation reaction occurs is provided in a region where the reaction heat generated in the hydrocarbon production step is transferred without passing through a heat medium.

3. The hydrocarbon production method according to claim 1, wherein a heat medium oil is used as a heat medium for utilizing the reaction heat generated in the hydrocarbon production step in the hydrogen extraction step.

4. The hydrocarbon production method according to claim 1, wherein water vapor is used as a heat medium for utilizing the reaction heat generated in the hydrocarbon production step in the hydrogen extraction step.

5. The hydrocarbon production method according to any one of claims 1 to 4, wherein the carbon monoxide is produced from carbon dioxide recovered from the atmosphere.

6. The hydrocarbon production method according to any one of claims 1 to 4, wherein the carbon monoxide is produced from carbon dioxide recovered from combustion exhaust gas.

7. The hydrocarbon production method according to claim 5 or 6, wherein the step of producing carbon monoxide is a reverse shift reaction step of producing the carbon monoxide from the carbon dioxide by a reverse shift reaction.

8. The hydrocarbon production method according to claim 7, wherein the reverse shift reaction step is performed at 290 to 340°C.

9. The hydrocarbon production method according to claim 7 or 8, wherein
in the reverse shift reaction step, a copper-based catalyst body containing a copper component composed of at least one of copper or copper oxide is used, and
in the reaction by the FT process, an iron-based catalyst body containing an iron component composed of at least one of iron or iron oxide and an added metal composed of at least one of alkali metal or alkaline earth metal is used.

10. The hydrocarbon production method according to claim 5 or 6, wherein the step of producing carbon monoxide is an electrolytic reduction step of producing the carbon monoxide from the carbon dioxide by electrolytic reduction.

11. The hydrocarbon production method according to any one of claims 1 to 10, wherein in the hydrocarbon production step, a reaction by the FT process occurs in an FT reaction vessel having a fixed bed.

12. The hydrocarbon production method according to any one of claims 1 to 10, wherein in the hydrocarbon production step, a reaction by the FT process occurs in an FT reaction vessel having a slurry bed.

13. The hydrocarbon production method according to any one of claims 1 to 12, wherein the hydrogen extraction step utilizes latent heat of water produced in the hydrocarbon production step.

14. The hydrocarbon production method according to any one of claims 1 to 13, wherein the hydrogen extraction step utilizes heat obtained by burning the hydrocarbon produced in the hydrocarbon production step.

15. The hydrocarbon production method according to any one of claims 1 to 14, wherein the hydrogen extraction step utilizes heat of an electric heater driven by electricity generated using the hydrocarbon produced in the hydrocarbon production step.
